# EUROPEAN PATENT APPLICATION

(11) **EP 3 088 028 A1**
(43) Date of publication of application: **02.11.2016**
(21) Application number: 14875487.2
(22) Date of filing: 10.11.2014
(51) Int. Cl.: A61M 5/32

(54) **CONNECTION STRUCTURE BETWEEN NEEDLE SEAT AND SYRINGE IN SAFE INJECTOR**

(30) Priority: 25.12.2013 CN 201310724627
(71) Applicant: Xu, Xuehua, Taizhou, Zhejiang 317605 (CN); Hangzhou Lanbishi Trade Co., Ltd., Chumen Town, Yuhuan County Taizhou Zhejiang 317605 (CN)
(72) Inventor: XU, Xuehua, Taizhou, Zhejiang 317605 (CN)
(74) Representative: Elzaburu S.L.P.
(86) International application number: PCT/CN2014/090686
(87) International publication number: WO 2015/096560

(57) **Abstract**

The invention provides a connecting structure for a syringe needle base and a syringe tube in a safety syringe, and belongs to the technical field of medical devices. The problem that the present self-destructing injector is poor in stability when destroying itself is solved. The connecting structure for the syringe needle base and the syringe tube in the safety syringe, characterized in that the front end of the syringe tube (1) is provided with a connecting tube (1 a) which is smaller than the outer diameter of the syringe tube (1); a syringe needle base (2) is in the shape of a column, and the front end of the syringe needle base (2) is used for being connected with the syringe needle (3); the connecting structure comprises elastic connecting pieces (2a) arranged at the rear end of the syringe needle base (2) and first connecting parts (1a1) arranged at the inner side of the connecting tube (1 a); the outer sides of the connecting pieces (2a) are provided with second connecting parts (2a1); the syringe needle base (2) is sleeved with the connecting tube (1 a); the connecting pieces (2a) are provided with an expanding part (5); the expanding part (5) is abutted against the inner side of the connecting pieces (2a) so that the outer sides of the connecting pieces (2a) are expanded to be in contact with the inner side of the connecting tube (1 a); under the expanding effect of the expanding part (5), the first connecting parts (1a1) are clamped with the second connecting parts (2a1). The connecting structure for the syringe needle base and the syringe tube in the safety syringe has the advantages of being high in stability and simple in structure.

## Description

### BACKGROUND OF THE INVENTION

### Field of Invention

The present invention relates to a safety syringe, in particular to a connecting structure for a syringe needle base and a syringe tube in a safety syringe.

### Related Art

In order to prevent the virus cross-infection caused by the use of unhygienic syringes, disposable syringes are widely used in the technical field of medical devices. Currently, in order to prevent some medical organizations, especially a small number of unregulated private medical clinics, from repeatedly using the disposable syringes for multiple times, the self-destructing function has been added to the disposable syringes.

In order to avoid using the syringes for the second time, and to prevent the staff from being stung after usage or during the process of destroying the syringes, which will lead to serious consequence of virus cross-infection, the safety syringes have been widely used in the medical field nowadays, and many countries have expressly stipulated in the medical laws and regulations that it is mandatory to use the safety syringes.

Taking a general view at all present retractable safety syringes, the fixing structure for the syringe needle base is mostly made by arranging a concave block or an annular groove inside the conical part of the syringe tube. Even if flanges are made on some syringes, this is only to emphasize the fact that the effect of the groove can be achieved. Then, it clamps with a protruding block or a protruding ring arranged outside the syringe needle base. By adopting this structural design, we should firstly consider the difficulty in developing the mould. Technically speaking, it is difficult to form a groove that is demanding a certain depth inside an empty barrel-shaped body such as the syringe tube. Even if the groove is formed somehow, its clamping strength may not be able to meet the requirement. Moreover, the clamping is achieved through the frictional force, so that clamping force of the groove is extremely likely to be affected by the variation of temperature. As a result, it is usually seen in the finished products that the syringe needle bases have already retracted back into the syringe tube before the packaging process, during the transportation process, or when using and installing the syringe needle, which makes the syringe can not be used normally, so great inconvenience is caused to the user. Due to the fact that by adopting only annular clamping, it is not likely to avoid the exerted rotational force when the syringe needle needs to be replaced, so the syringe needle base usually rotates along with the syringe needle. The syringe needle base cannot be firmly assembled with the syringe needle, and the syringe needle cannot be detached from the syringe needle base as well, thus causing great inconvenience to the user. So in order to improve this condition, designers will then have to additionally arrange rotation-limiting devices on the syringe tube and the syringe needle base, which greatly increases the cost for mould development and the difficulty in injection moulding and quality inspection.

Take the safety self-destructing syringe (Application number: 201010202077.9; Publication number: CN101850144A) disclosed in the Chinese patent literature as an example. It comprises a syringe tube, a syringe rod, a rubber plug, and a cylinder-shaped syringe needle base. The rubber plug is fixedly connected with the front of the syringe rod. The syringe needle base is connected to the inner side at the front of the syringe tube. The front of the syringe rod is provided with a rod head which penetrates through the rubber plug and extends out of the rubber plug. The end of the rod head is provided with a positioning part. The inner wall of the syringe needle base is provided with a protruding body. A clamping structure is arranged between the positioning part of the rod head and the protruding body. When the rod head is inserted into the syringe needle base, the positioning part is capable of preventing the rod head from exiting the syringe needle base by utilizing the elasticity of the positioning part itself or the protruding body in combination with the protruding body. Once the injection is completed by using this safety self-destructing syringe, then the medical worker continuously pushes the syringe rod in the injecting direction. Due to the fact that the rubber plug is elastic, and the rubber plug is squeezed while the positioning part is continuously moved forward, the positioning part or the protruding body will be bent during the moving process. When the positioning part passes through the protruding body, the bent positioning part or protruding body reset under the effect of the elastic force. The reset positioning part is blocked by the protruding body, forcing the positioning part to be clamped inside the syringe needle base. Then the syringe rod is retreated to pull the syringe needle base back into the syringe tube, and finally the syringe rod is broken, therefore the purpose of self-destruction is achieved.

The above safety self-destructing syringe can achieve safe self-destruction after being withdrawn from the recipient's body, thus preventing the damage to the human body caused by the self-destruction. However, due to the fact that the syringe needle base is fixedly connected with the syringe tube directly in a close fit mode, once the close fit loses its efficacy, the syringe needle base may rotate or even move relative to the syringe needle base. Therefore, the stability is poor. Other prior art safety syringes share the similar problems.

### SUMMARY OF THE INVENTION

One objective of a preferred embodiment of the invention is to resolve the above problems in the prior art, and to provide a connecting structure for a syringe needle base and a syringe tube in a safety syringe, which is high in stability and simple in structure.

One objective of a preferred embodiment of the invention can be achieved through the following technical schemes: a connecting structure for a syringe needle base and a syringe tube in a safety syringe, characterized in that the front end of the syringe tube is provided with a connecting tube which is smaller than the outer diameter of the syringe tube; a syringe needle base is in the shape of a column, and the front end of the syringe needle base is used for being connected with the syringe needle; the connecting structure comprises elastic connecting pieces arranged at the rear end of the syringe needle base and first connecting parts arranged at the inner side of the connecting tube; the outer sides of the connecting pieces are provided with second connecting parts; the syringe needle base is sleeved with the connecting tube; the inner sides of the connecting pieces are provided with an expanding part; the expanding part is abutted against the inner sides of the connecting pieces so that the outer sides of the connecting pieces are expanded to be in contact with the inner side of the connecting tube; under the expanding effect of the expanding part, the first connecting parts are clamped with the second connecting parts.

When the first connecting part and the second connecting part in this structure are clamped with each other, the syringe needle base can be connected with the connecting tube. Due to the fact that the first connecting parts are clamped with the second connecting parts, connecting the syringe needle base with the connecting tube can further prevent the syringe needle base from rotating relative to the connecting tube.

Of course, when the safety syringe is used normally, the expanding part is always abutted against the connecting pieces, so it is guaranteed that the first connecting parts are clamped with the second connecting parts together all the time. During the self-destruction process, a pushing rod of the syringe will first drive the expanding part to be detached from the syringe needle base, and then the connecting pieces of the syringe needle base retract due to the elastic force of their own so that the first connecting parts are detached from the second connecting parts. At this moment, the syringe needle base can only be pulled into the syringe tube by continuously pulling the pushing rod downward.

The above connecting structure for the syringe needle base and the syringe tube in the safety syringe, characterized in that the first connecting parts are protruding blocks which protrude out of the inner side of the connecting tube, and the second connecting parts are grooves which sink into the outer side of the connecting pieces and are matched with the protruding blocks.

After the protruding blocks are embedded into the grooves, the syringe needle base can be fixedly connected to the inside of the connecting tube in the circumferential direction and the axial direction.

The above connecting structure for the syringe needle base and the syringe tube in the safety syringe, characterized in that the number of the protruding blocks is two, and the two protruding blocks are symmetrically distributed on the two sides in the connecting tube; the number of the connecting pieces is two, and each connecting piece is provided with one groove.

By symmetrically arranging the two protruding blocks, the connecting stability can be improved, the stress is even after being connected, and the stability is relatively high.

The above connecting structure for the syringe needle base and the syringe tube in the safety syringe, characterized in that the upper edges, facing the end of the connecting tube, of the protruding blocks are provided with first inclined faces; the upper edges of the grooves in the connecting pieces are also provided with second inclined faces, and the direction of inclination of the second inclined faces is the same as that of the first inclined faces of the upper edges of the protruding blocks.

During the self-destruction process of the safety syringe, the expanding part is firstly pulled downward through the pushing rod, and the expanding part moves after being pulled downward so that the expanding effect exerted on the connecting pieces is eliminated. At this moment, the first connecting parts are detached from the second connecting parts. Then the pushing rod which is continuously being pulled downward drives the syringe needle base to move downward, and finally the syringe needle base is pulled into the syringe tube.

Serving as another scheme, the above connecting structure for the syringe needle base and the syringe tube in the safety syringe, characterized in that the first connecting parts are the grooves which sink into the inner side of the connecting tube, and the second connecting parts are the protruding blocks which protrude out of the outer side of the connecting pieces and are matched with the grooves.

Similarly, the syringe needle base can also be fixedly connected to the inside of the connecting tube in the circumferential direction and the axial direction through the combination of the grooves and the protruding blocks.

The above connecting structure for the syringe needle base and the syringe tube in the safety syringe, characterized in that the expanding part is in the shape of a cylinder; a limiting structure which is capable of moving the expanding part up and down within the set range is arranged between the upper end of the expanding part and the connecting pieces; the outer side at the lower end of the expanding part is provided with a pushing part which is used for expanding the connecting pieces towards the inner side of the connecting tube; the outer diameter of the pushing part is greater than the size between the two connecting pieces.

When the safety syringe is used normally, the pushing part of the expanding part is abutted against the inner side of the connecting pieces. Due to the fact that the size of the pushing part is large, the connecting pieces will press against the inner side the connecting tube after deforming elastically, and finally the first clamping part is clamped with the second clamping part.

The limiting structure can enable the expanding part to move up and down within the set range in the axial direction of the syringe needle base, so that the expanding part is prevented from being detached from the syringe needle base during the self-destruction process of the safety syringe, which affects the normal self-destruction of the syringe.

The above connecting structure for the syringe needle base and the syringe tube in the safety syringe, characterized in that an inclined guiding face is arranged between the pushing part and the expanding part.

Due to the fact that the size of the pushing part is large, the pushing part can be smoothly moved into the syringe needle base through the guiding face, and the connecting pieces are expanded after moving the expanding part.

The above connecting structure for the syringe needle base and the syringe tube in the safety syringe, characterized in that the limiting structure comprises a convex annular first protruding ring arranged at the inner side of the connecting pieces and a convex annular second protruding ring arranged at the outer side of the expanding part; the second protruding ring is positioned between the end of the syringe needle base and the first protruding ring, and the first protruding ring can be abutted against the second protruding ring.

By adopting the combination of the first protruding ring and the second protruding ring, the pushing part of the expanding part can be detached from the connecting pieces, while it can be guaranteed that the expanding part will not be detached from the syringe needle base.

The above connecting structure for the syringe needle base and the syringe tube in the safety syringe, characterized in that a sealing ring is arranged between the syringe needle base and the syringe tube.

The sealing performance between the syringe needle base and the connecting tube is improved by the sealing ring.

The above connecting structure for the syringe needle base and the syringe tube in the safety syringe, characterized in that the connecting pieces are arranged in an inclined mode; a gap exists between the syringe needle base and the connecting tube; the second connecting parts are close to the ends of the connecting pieces; the outer side at the middle of the syringe needle base is provided with a sealing ring groove; the sealing ring is positioned in the sealing ring groove, and the sealing ring is tightly pressed between the syringe needle base and the connecting tube.

A gap exists between the syringe needle base and the connecting tube; the position where the syringe needle base is connected with the connecting tube comprises the sealing ring arranged between the two, and the connection between the two connecting pieces and the connecting tube. It is guaranteed that the connecting tube can be firmly connected with the syringe needle base even with a small contact area. Meanwhile, as long as the expanding part is moved downward, after the expanding force exerted onto the connecting pieces is relieved, the syringe needle base can still be smoothly detached from the connecting tube.

The above connecting structure for the syringe needle base and the syringe tube in the safety syringe, characterized in that the annular first connecting parts protrude out of the inner side of the connecting tube; the connecting pieces are matched with the inner side of the connecting tube; the second connecting parts are arc-shaped grooves which sink into the connecting pieces.

Due to the fact that the first connecting parts protrude in an annular mode, so the contact area between the first connecting parts and the second connecting parts is relatively large, thus guaranteeing the connecting stability.

The above connecting structure for the syringe needle base and the syringe tube in the safety syringe, characterized in that the syringe needle base comprises a first base body and a second base body; the first base body is used for being connected with the syringe needle; the connecting pieces are positioned on the second base body; the outer side at the lower end of the first base body is provided with a concave ring-shaped connecting groove; the second base body is in the shape of a barrel, and the inner side of the second base body is provided with a protruding ring-shaped connecting ring; the first base body is sleeved with the second base body, and the connecting ring is embedded into the connecting groove under the expanding force of an expanding ring.

The syringe needle base is of the split-type structure, so the whole syringe needle base is convenient to process.

The above connecting structure for the syringe needle base and the syringe tube in the safety syringe, characterized in that the concave ring-shaped sealing ring groove is formed between the first base body and the outer side of the second base body.

Meanwhile, the sealing ring can be positioned after the first base body is connected with the second base body, so it is not necessary to form additional sealing ring groove in the syringe needle base. The structure of the syringe needle base is simplified, and the production cost is lowered.

Compared with the prior art, in the connecting structure for the syringe needle base and the syringe tube in the safety syringe, due to the fact that the protruding blocks of the connecting tube are matched with the grooves in the elastic connecting pieces, the syringe needle base and the connecting tube connected together can be fixedly connected in not only the circumferential direction, but also the axial direction. It can be seen that when assembling the syringe provided with this structure, due to the fact that the syringe needle base does not rotate relative to the connecting tube, so the syringe is convenient to assemble and the assembling efficiency is high.

Meanwhile, when the safety syringe is used normally, due to the fact the pushing part of the expanding part is always abutted against the inner side of the connecting pieces, as long as the pushing part of the expanding part does not detach from the connecting pieces, then the protruding blocks is always clamped with the grooves. This guarantees that the syringe needle does not retract into the syringe tube when using the syringe normally, and the in-use stability of the safety syringe is improved.

In addition, due to the fact that the syringe needle base is of the split-type structure, the first base body can be firstly sleeved with the sealing ring, and then the second base body can be connected with the first base body. Therefore, the sealing ring is convenient to install.

### BRIEF DESCRIPTION OF THE DRAWINGS

FIG. 1 is a sectional view of a preferred embodiment of the connecting structure for the syringe needle base and the syringe tube in the safety syringe during the self-destruction process.
FIG. 2 is a partial sectional view of the connecting tube and the syringe needle base in FIG. 1.
FIG. 3 is a sectional view of a preferred embodiment of the connecting structure for the syringe needle base and the syringe tube in the safety syringe after the self-destruction process.
FIG. 4 is a sectional view of a preferred embodiment of the connecting tube in the connecting structure for the syringe needle base and the syringe tube in the safety syringe.
FIG. 5 is a sectional view of Section A-A in FIG. 4.
FIG. 6 is a sectional view of a preferred embodiment of the syringe needle base in the connecting structure for the syringe needle base and the syringe tube in the safety syringe.
FIG. 7 is a sectional view of a preferred embodiment of the first base body in the connecting structure for the syringe needle base and the syringe tube in the safety syringe.
FIG. 8 is a sectional view of a preferred embodiment of the second base body in the connecting structure for the syringe needle base and the syringe tube in the safety syringe.
FIG. 9 is a sectional view of a preferred embodiment of the syringe needle base in the connecting structure for the syringe needle base and the syringe tube.
FIG. 10 is a sectional view of a preferred embodiment of the expanding part in the connecting structure for the syringe needle base and the syringe tube.
FIG. 11 is a sectional view of a preferred embodiment of the connecting structure for the syringe needle base and the syringe tube in the second embodiment.
FIG. 12 is a perspective view of the syringe needle base in FIG. 11.
FIG. 13 is a sectional view of the connecting tube in FIG. 11.
FIG. 14 is an exploded view of the connecting structure for the syringe needle base and the syringe tube in the first embodiment.
FIG. 15 is an exploded view of the connecting structure for the syringe needle base and the syringe tube in the second embodiment.
FIG. 16 is a sectional view of the initial state of the connecting structure for the syringe needle base and the syringe tube.
FIG. 17 is an enlarged view in FIG. 14.
FIG. 18 is an enlarged view in FIG. 15.

### DETAILED DESCRIPTION OF THE INVENTION

### First Embodiment

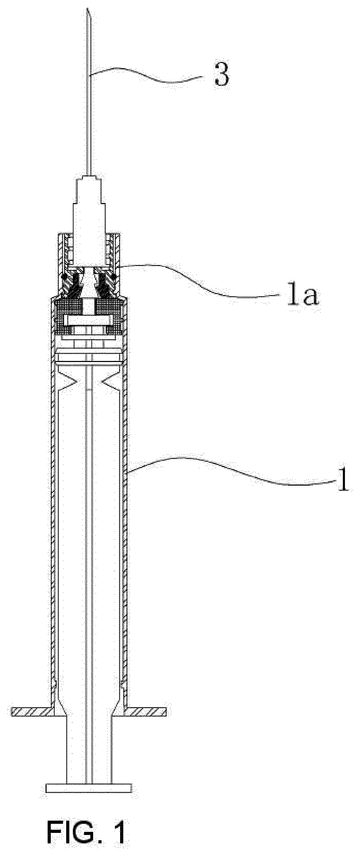

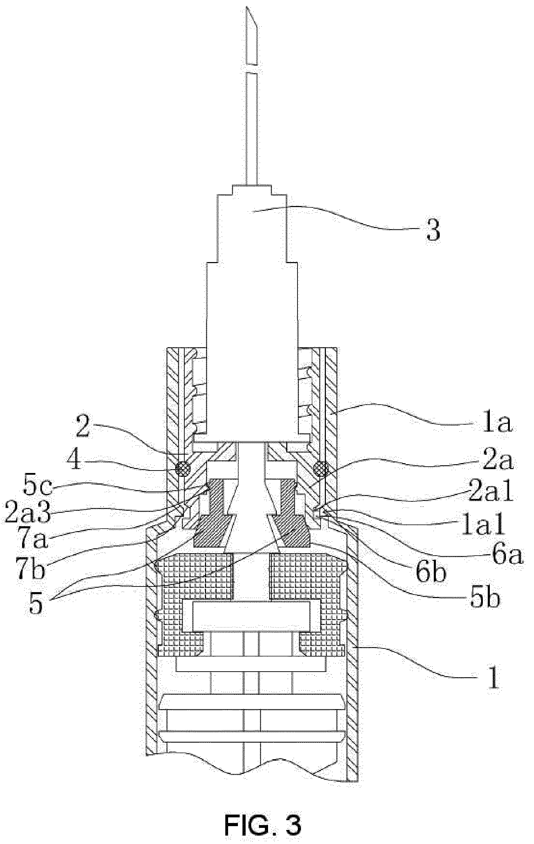

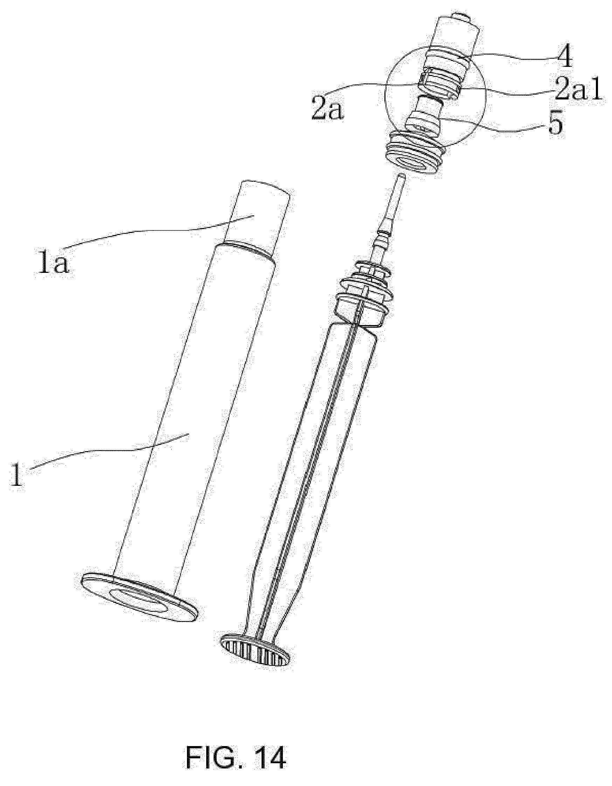

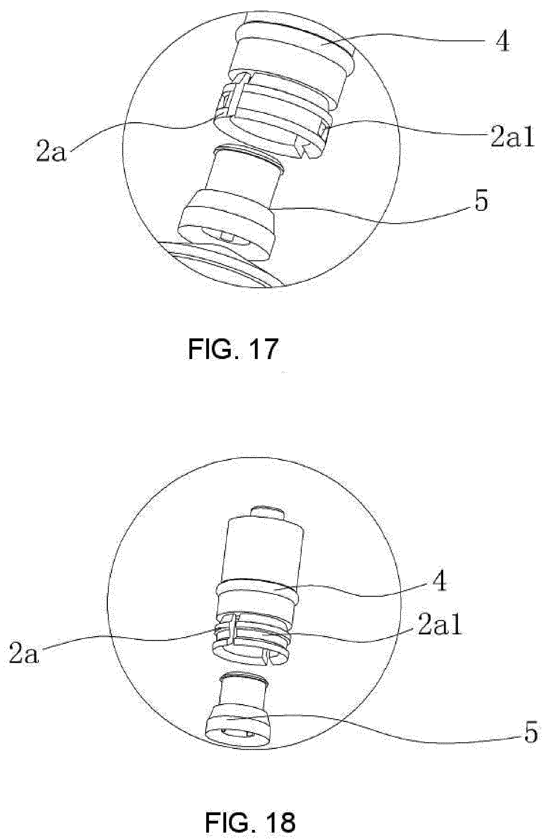

In a first preferred embodiment as shown in FIGs.1, 2, 3, 14, and 17, the front end of the syringe tube (1) of the safety syringe is provided with the connecting tube (1 a); the outer diameter of the connecting tube (1a) is slightly smaller than that of the syringe tube (1); the front end of the syringe needle base (2) is connected to the syringe needle (3). The syringe needle base (2) is sleeved with the connecting tube (1 a), and the two are connected with each other.

The connecting structure for the syringe needle base and the syringe tube comprises the elastic connecting pieces (2a) arranged at the rear end of the syringe needle base (2) and first connecting parts (1a1) arranged at the inner side of the connecting tube (1a); the outer sides of the connecting pieces (2a) are provided with the second connecting parts (2a1); the syringe needle base (2) is sleeved with the connecting tube (1 a), and the first connecting parts (1a1) are clamped with the second connecting parts (2a1).

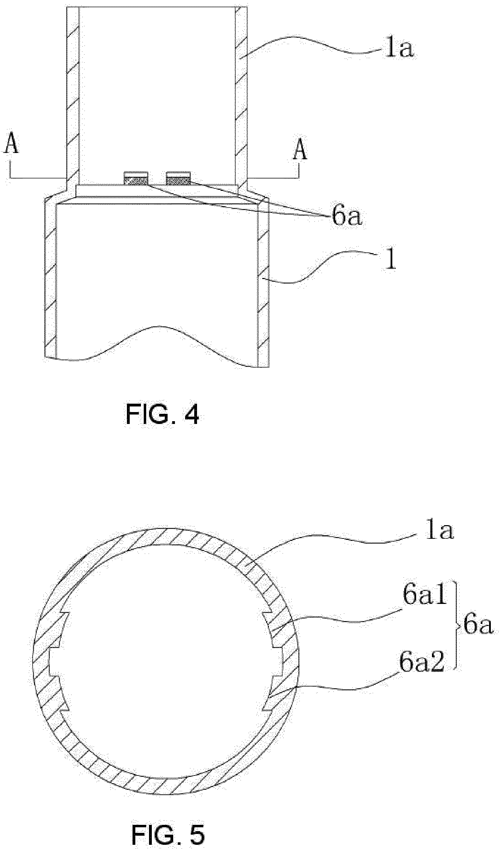

In this embodiment, as shown in FIGs.4 and 5, the first connecting parts (1a1) are the protruding blocks (6a) which protrude out of the inner side of the connecting tube (1a); the second connecting parts (2a1) are grooves (6b) which sink into the outer side of the connecting pieces (2a); the grooves (6b) are matched with the protruding blocks (6a), and the protruding blocks (6a) are embedded into the grooves (6b). Based on the actual condition, the positions of the protruding blocks (6a) and the grooves (6b) can be swapped, in another word, the first connecting parts (1a1) are the grooves which sink into the inner side of the connecting tube (1a), and the second connecting parts (2a1) are the protruding blocks (6a) which protrude out of the outer side of the connecting pieces (2a) and are matched with the grooves (6b).

The number of the protruding blocks (6a) is two, and the two protruding blocks (6a) are symmetrically distributed on the two sides in the connecting tube (1a); the number of the connecting pieces (2a) is two, and each connecting piece (2a) is provided with one groove (6b); the protruding blocks (6a) are matched with the grooves (6b) in a one-to-one corresponding mode. In this embodiment, Each protruding block (6a) is formed by adjacent first protruding block (6a1) and second protruding block (6a2), in another word, four connecting points are provided for the positions where the syringe needle base (2) are connected with the connecting tube (1 a). Of course, based on the actual condition, the number of minor protruding blocks on each protruding block (6a) can be increased, and the number of grooves on the corresponding connecting piece (2a) can be increased as well.

The upper edges, facing the end of the connecting tube (1 a), of the protruding blocks (6a) are first inclined faces (7a); the upper edges of the grooves (6b) in the connecting pieces (2a) are second inclined faces (7b); the direction of inclination of the second inclined faces (7b) of the upper edges of the grooves (6b) is the same as that of the first inclined faces (7a) of the upper edges of the protruding blocks (6a) and the angles of inclination are the same as well.

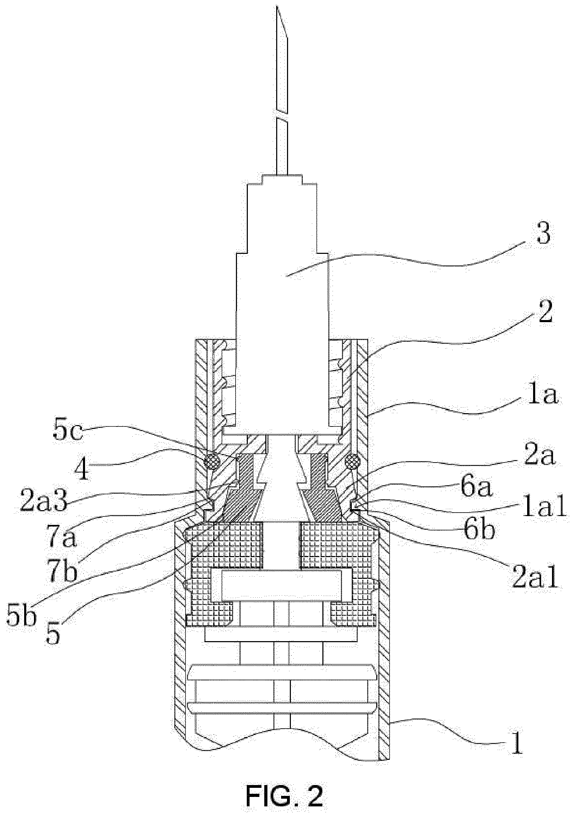

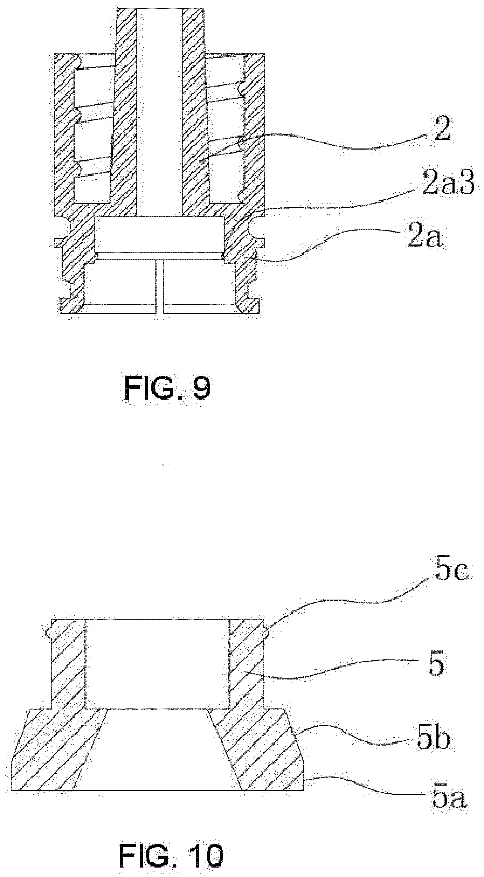

As shown in FIGs. 2 and 10, the syringe needle base (2) is provided with a cylinder-shaped expanding part (5); a limiting structure which is capable of moving the expanding part (5) up and down within the set range is arranged between the upper end of the expanding part (5) and the connecting pieces (2a); the outer side at the lower end of the expanding part (5) is provided with a pushing part (5a) which is used for expanding the connecting pieces (2a) towards the inner side of the connecting tube (1 a); the outer diameter of the pushing part (5a) is greater than the size between the two connecting pieces (2a). In this embodiment, an inclined guiding face (5b) is arranged between the pushing part (5a) and the expanding part (5).

The limiting structure comprises a convex annular first protruding ring (2a3) arranged at the inner side of the connecting pieces (2a) and a convex annular second protruding ring (5c) arranged at the outer side of the expanding part (5); the second protruding ring (5c) is positioned between the end of the syringe needle base (2) and the first protruding ring (2a3), and the first protruding ring (2a3) can be abutted against the second protruding ring (5c). Of course, based on the actual condition, another scheme can be adopted to the limiting structure as well, namely, the limiting structure comprises limiting grooves which sink into the inner side of the connecting pieces (2a) and inserting rods which protrude out of the outer side of the expanding part (5); the inserting rods are positioned in the limiting grooves.

In order to improve the sealing performance, a sealing ring (4) is arranged between the syringe needle base (2) and the syringe tube (1).

When the expanding part (5) is positioned in the connecting pieces (2a) and exerts the outward expanding force onto the connecting pieces (2a), the connecting pieces (2a) are in an outward inclined state, the ends of the connecting pieces (2a) partially protrude out of the outer side of the syringe needle base (2). A gap exists between the syringe needle base (2) and the connecting tube (1 a), and the second connecting parts (2a1) are close to the ends of the connecting pieces (2a). The outer side at the middle of the syringe needle base (2) is provided with a sealing ring groove (2a2); the sealing ring (4) is positioned in the sealing ring groove (2a2), and the sealing ring (4) is tightly pressed between the syringe needle base (2) and the connecting tube (1 a).

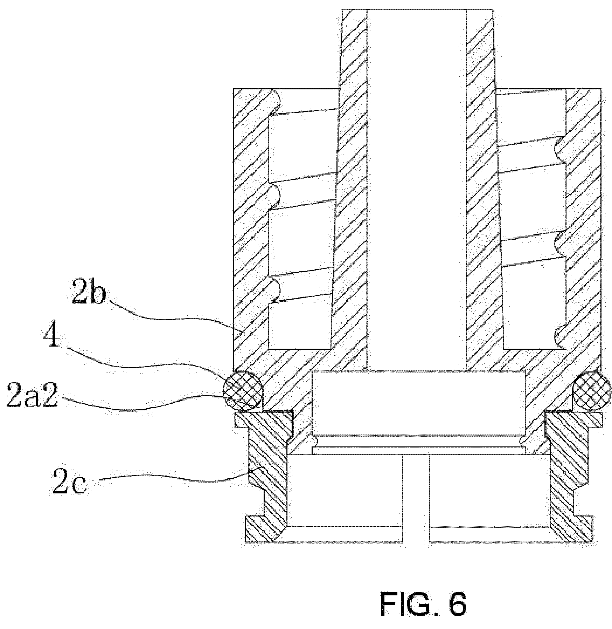

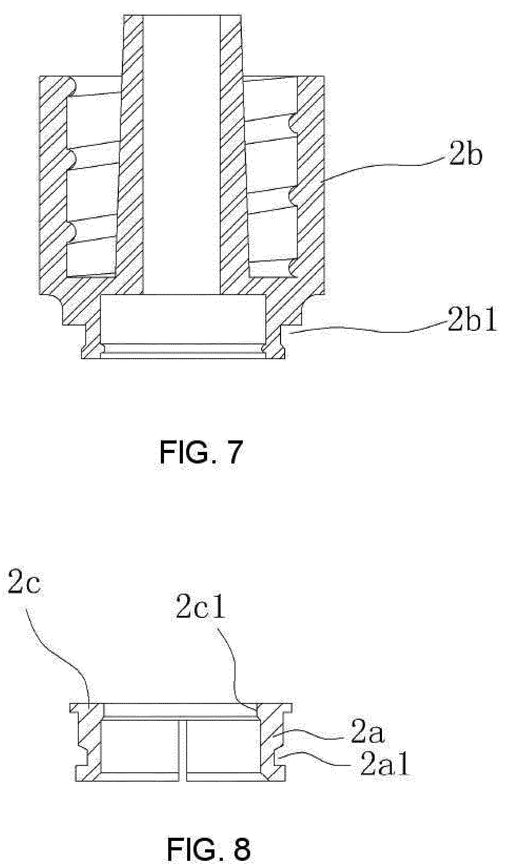

In this embodiment, the syringe needle base (2) comprises a first base body (2b) and a second base body (2c); the first base body (2b) is connected with the syringe needle (3); the connecting pieces (2a) are positioned at the end of the second base body (2c). the outer side at the lower end of the first base body (2b) is provided with a concave ring-shaped connecting groove (2b1); the second base body (2c) is in the shape of a barrel, and the inner side of the second base body (2c) is provided with a protruding ring-shaped connecting ring (2c1); the first base body (2b) is sleeved with the second base body (2c), and the connecting ring (2c1) is embedded into the connecting groove (2b1). The concave ring-shaped sealing ring groove (2a2) is formed between the first base body (2b) and the outer side of the second base body (2c); the sealing ring (4) is positioned in the sealing ring groove (2a2), see FIGs.6, 7, and 8.

When the first connecting part (1a1) and the second connecting part (2a1) in this structure are clamped with each other, the syringe needle base (2) can be connected with the connecting tube (1 a). Due to the fact that the first connecting parts (1a1) are clamped with the second connecting parts (2a1), connecting the syringe needle base (2) with the connecting tube (1a) can further prevent the syringe needle base (2) from rotating relative to the connecting tube (1a). It can be seen that when the syringe needle (3) is connected to the syringe needle base (2), due to the fact that the syringe needle base (2) dose not rotate, the syringe needle (3) can be conveniently connected to the syringe needle base (2) by applying the rotational force, and the syringe needle (3) of the syringe is firmly connected with the syringe needle base (2) after the assembling is completed.

When the safety syringe provided with this structure is used normally, the pushing part (5a) of the expanding part (5) is abutted against the inner side of the connecting pieces (2a); the outer sides of the connecting pieces (2a) are abutted against the inner side of the connecting tube (1 a) under the expanding effect of the expanding part (5), and finally the first connecting parts (1a1) are firmly clamped with the second connecting parts (2a1). At this moment, due to the fact that the expanding part (5) is not detached from the syringe needle base (2), the syringe needle base (2) does not retract into the syringe tube (1) when the syringe is used normally.

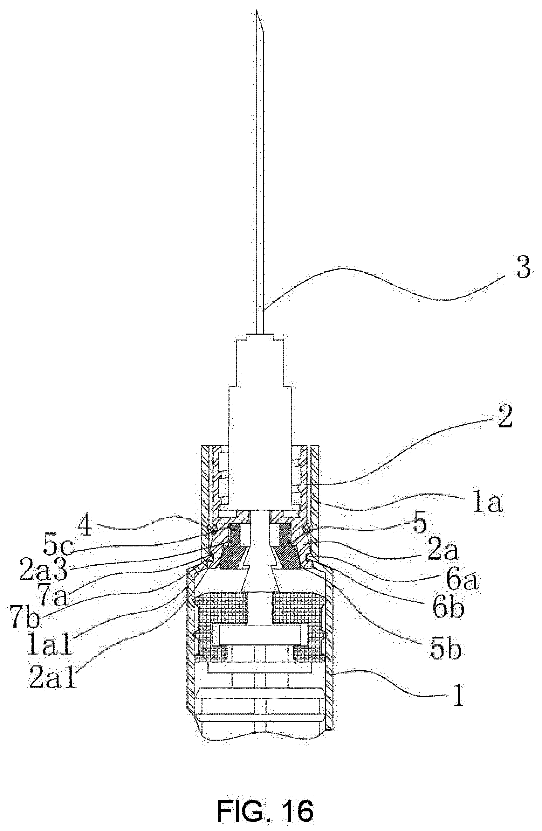

As shown in FIGs. 2, 3, and 16, when the safety syringe is in the initial state, the syringe needle base (2) is positioned at the end of the syringe tube (1); the syringe needle (3) is connected to the inside of the syringe needle base (2) in a threaded mode; the expanding part (5) is positioned at the inner sides of the connecting pieces (2a); the syringe needle base (2) enables the connecting pieces (2a) to be clamped with the connecting tube (1a) through the effect of the expanding part (5) acted onto the connecting pieces (2a); therefore, the syringe needle base (2) is connected with the syringe tube (1). At this moment, the end of a piston rod is yet to enter the expanding part (5); the piston rod can be pulled backward to draw liquid; the injection can be completed by pushing the piston rod forward, and then the front end of the piston rod can penetrate through the expanding part (5) by continuously pushing the piston rod; a connection relation is formed through the expanding part (5) and the stepped face at the front end of the piston rod. During the self-destruction process of the safety syringe, a pushing rod of the piston rod pulls the expanding part (5) to move downward through a pulling part formed by the stepped face; after the expanding part (5) is moved downward, the pushing part (5a) of the expanding part (5) is detached from the inner sides of the connecting pieces (2a); the connecting pieces (2a) retract under the effect of their own elastic force, and the outer sides of the connecting pieces (2a) are detached from the connecting tube (1a). Of course, the first connecting parts (1a1) are also detached from the second connecting parts (2a1) at this moment. Under the effect of the limiting structure, the expanding part (5) which is moved downward will not be detached from the syringe needle base (2), and after pulling the pushing rod continuously, the syringe needle base (2) is finally pulled into the syringe tube (1) so that the self-destruction is achieved.

### Second Embodiment

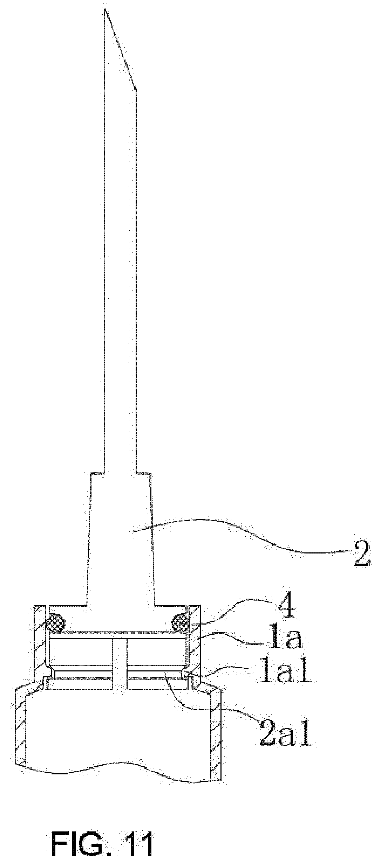

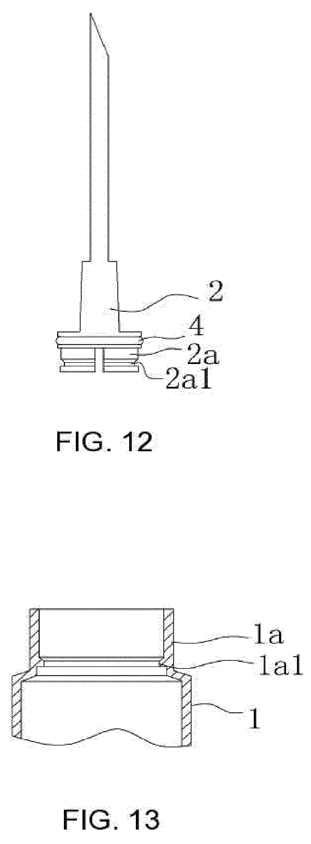

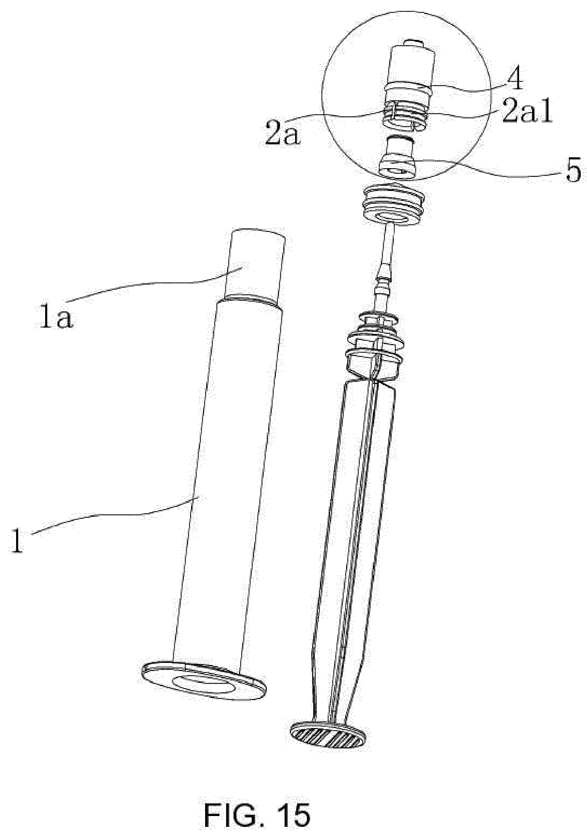

This second preferred embodiment is essentially identical to the first embodiment in terms of the structure and principles, but the differences are: the annular first connecting parts (1a1) protrude out of the inner side of the connecting tube (1 a); the connecting piece (2a) is in the shape of an arc which is matched with the inner side of the connecting tube (1 a); the second connecting parts (2a1) are arc-shaped grooves which sink into the outer sides connecting pieces (2a), see FIGs.11, 12, 13,15, and 18. The number of the connecting pieces (2a) in this embodiment can be two or more.

### Third Embodiment

As shown in FIGs.9, 11, 12, and 13, this third preferred embodiment is essentially identical to the first embodiment or the second embodiment in terms of the structure and principles, but the differences are: in this embodiment, the syringe needle base (2) is integrally formed; the syringe needle base (2) and the syringe needle (3) are all connected as a whole; the connecting pieces (2a) are positioned at the rear end of the syringe needle base (2); the outer side of the syringe needle base (2) is provided with a concave ring-shaped sealing ring groove (2a2); the sealing ring (4) is embedded into the sealing ring groove (2a2).

The description of the preferred embodiments thereof serves only as an illustration of the spirit of the invention. Various modifications, or supplements, or adoption of similar methods as alternatives may be made therein by those skilled in the art without departing from the spirit of the invention or exceeding the scope defined in the appended Claims.

### List of Reference Numerals

- 1: syringe tube
- 1 a: connecting tube
- 1a1: first connecting part
- 2: syringe needle base
- 2a: connecting piece
- 2a1: second connecting part
- 2a2: sealing ring groove
- 2a3: first protruding ring
- 2b: first base body
- 2b1: connecting groove
- 2c: second base body
- 2c1: connecting ring
- 3: syringe needle
- 4: sealing ring
- 5: expanding part
- 5a: pushing part
- 5b: guiding face
- 5c: second protruding ring
- 6a: protruding block
- 6a1: first protruding block
- 6a2: second protruding block
- 6b: groove
- 7a: first inclined face
- 7b: second inclined face

## Claims

1. A connecting structure for a syringe needle base and a syringe tube in a safety syringe, comprising:
a front end of the syringe tube (1) provided with a connecting tube (1 a) that is smaller than the outer diameter of the syringe tube (1);
a syringe needle base (2) in a shape of a column;
a front end of the syringe needle base (2) used for connecting with a syringe needle (3);
elastic connecting pieces (2a) of the connecting structure arranged at a rear end of the syringe needle base (2);
first connecting parts (1a1) arranged at an inner side of the connecting tube (1 a);
outer sides of the connecting pieces (2a) provided with second connecting parts (2a1); and
inner sides of the connecting pieces (2a) provided with an expanding part (5);
wherein the syringe needle base (2) is sleeved with the connecting tube (1 a);
wherein the expanding part (5) is abutted against the inner sides of the connecting pieces (2a) so that the outer sides of the connecting pieces (2a) are expanded to be in contact with the inner side of the connecting tube (1 a); and
wherein under an expanding effect of the expanding part (5), the first connecting parts (1a1) are clamped with the second connecting parts (2a1).

2. A connecting structure as claimed in the Claim 1, wherein the first connecting parts (1 a1) are protruding blocks (6a) that protrude out of the inner side of the connecting tube (1 a), and the second connecting parts (2a1) are grooves (6b) that sink into the outer side of the connecting pieces (2a) and are matched with the protruding blocks (6a).

3. A connecting structure as claimed in the Claim 2, wherein there are two protruding blocks (6a), and the two protruding blocks (6a) are symmetrically distributed on two sides in the connecting tube (1 a);
Wherein there are two connecting pieces (2a), and the outer side of each connecting piece (2a) is provided with one groove (6b); and
wherein the expanding part (5) is positioned between the two connecting pieces (2a).

4. A connecting structure as claimed in the Claim 3, wherein upper edges, facing an end of the connecting tube (1 a), of the protruding blocks (6a) are provided with first inclined faces (7a);
Wherein upper edges of the grooves (6b) in the connecting pieces (2a) are provided with second inclined faces (7b); and
wherein a direction of inclination of the second inclined faces (7b) is the same as a direction of inclination of the first inclined faces (7a) of the upper edges of the protruding blocks (6a).

5. A connecting structure as claimed in the Claim 1, wherein the first connecting parts (1 a1) are the grooves that sink into an inner side of the connecting tube (1a), and the second connecting parts (2a1) are the protruding blocks that protrude out of an outer side of the connecting pieces (2a) and are matched with the grooves.

6. A connecting structure as claimed in the Claim 1, 2, 3, 4 or 5, wherein the expanding part (5) is in a shape of a cylinder;
wherein a limiting structure that is capable of moving the expanding part (5) up and down within a set range is arranged between an upper end of the expanding part (5) and the connecting pieces (2a);
whereinan outer side at a lower end of the expanding part (5) is provided with a pushing part (5a) thatis capable of expanding the connecting pieces (2a) towards an inner side of the connecting tube (1 a); and
wherein an outer diameter of the pushing part (5a) is greater than a distance between the two connecting pieces (2a).

7. A connecting structure as claimed in the Claim 6, wherein an inclined guiding face (5b) is arranged between the pushing part (5a) and a middle of the expanding part (5).

8. A connecting structure as claimed in the Claim 6, wherein the limiting structure comprises a convex annular first protruding ring (2a3) arranged at an inner side of the connecting pieces (2a) and a convex annular second protruding ring (5c) arranged at an outer side of the expanding part (5);
Wherein the second protruding ring (5c) is positioned between an end of the syringe needle base (2) and the first protruding ring (2a3); and
Wherein the first protruding ring (2a3) is capable of being abutted against the second protruding ring (5c).

9. A connecting structure as claimed in the Claim 1, wherein the annular first connecting parts (1a1) protrude out of an inner side of the connecting tube (1 a);
wherein the connecting pieces (2a) are matched with an inner side of the connecting tube (1 a); and
wherein the second connecting parts (2a1) are arc-shaped grooves that sink into the connecting pieces (2a).

10. A connecting structure as claimed in the Claim 1, 2, 3, 4 or 5, wherein the syringe needle base (2) comprises a first base body (2b) and a second base body (2c);
wherein the first base body (2b) is capable of connecting with the syringe needle (3);
wherein the connecting pieces (2a) are positioned on the second base body (2c);
wherein an outer side at a lower end of the first base body (2b) is provided with a concave ring-shaped connecting groove (2b1);
wherein the second base body (2c) is in a shape of a barrel, and an inner side of the second base body (2c) is provided with a protruding ring-shaped connecting ring (2c1);
wherein the first base body (2b) is sleeved with the second base body (2c); and
wherein the connecting ring (2c1) is embedded into the connecting groove (2b1).

11. A connecting structure as claimed in the Claim 1, 2, 3, 4 or 5, wherein the syringe needle base (2) is connected with the syringe needle (3) as a whole;
wherein the connecting pieces (2a) are positioned at a rear end of the syringe needle base (2);
wherein an outer side of the syringe needle base (2) is provided with a concave ring-shaped sealing ring groove (2a2); and
wherein a sealing ring (4) is embedded into the sealing ring groove (2a2).
